# EUROPÄISCHE PATENTSCHRIFT

(11) **EP 3 201 169 B1**
(45) Veröffentlichungstag und Bekanntmachung des Hinweises auf die Patenterteilung: **03.06.2020**
(21) Anmeldenummer: 15770925.4
(22) Anmeldetag: 29.09.2015
(51) Int. Cl.: C07C 201/08, C07C 205/06, B01D 53/00

(54) **ABGASREINIGUNG IN EINEM VERFAHREN ZUR KONTINUIERLICHEN HERSTELLUNG VON DINITROTOLUOL**
EXHAUST GAS PURIFICATION IN A METHOD FOR THE CONTINUOUS PRODUCTION OF DINITROTOLUENE
NETTOYAGE DE GAZ D'ÉCHAPPEMENT DANS UN PROCÉDÉ DE FABRICATION CONTINUE DE DINITROTOLUÈNE

(30) Priorität: 02.10.2014 EP 14187532
(43) Veröffentlichungstag der Anmeldung: 09.08.2017
(73) Patentinhaber: Covestro Deutschland AG, 51373 Leverkusen (DE)
(72) Erfinder: KNAUF, Thomas, 41542 Dormagen (DE); MÜNNIG, Jürgen, 41564 Kaarst (DE); LORENZ, Wolfgang, 41540 Dormagen (DE); PENNEMANN, Bernd, 51467 Bergisch Gladbach (DE)
(74) Vertreter: Levpat
(86) Internationale Anmeldenummer: PCT/EP2015/072397
(87) Internationale Veröffentlichungsnummer: WO 2016/050759

(56) Entgegenhaltungen:
- EP-A1- 0 155 586
- EP-A1- 1 496 043
- EP-A1- 1 880 989
- EP-A1- 2 719 682
- WO-A1-2011/082977
- WO-A1-2014/016290

## Beschreibung

Die vorliegende Erfindung betrifft ein Verfahren zur Herstellung von Dinitrotoluol, umfassend die Schritte:
a) Nitrierung von Toluol mit einem Gemisch aus Salpetersäure und Schwefelsäure mit anschließender Abtrennung einer bei der Nitrierung entstehenden Schwefelsäure-haltigen wässrigen Phase, wobei ein Roh-Dinitrotoluol erhalten wird,
b) Waschen des Roh-Dinitrotoluols in einer Wasserwäsche mit neutralem und/oder alkalischem Waschwasser, wobei nach Abtrennung des in der letzten Wäsche eingesetzten Waschwassers ein vorgereinigtes Dinitrotoluol erhalten wird, welches neben Dinitrotoluol zumindest Wasser enthält und
c) Abtrennen des Wassers aus dem vorgereinigten Dinitrotoluol,
d) Sammeln der Abwässer aus den Schritten a), b) und/oder c),
e) optional, Extraktion der gesammelten Abwässer aus Schritt d) mit Toluol und Rückführung der so erhaltenen organischen Phase in Schritt a),
f) Befreiung der gesammelten Abwässer aus Schritt d), bzw., falls der optionale Schritt e) durchgeführt wird, des extrahierten Abwassers aus Schritt e), von Toluol in einem Toluolstripper, wobei ein Toluol-haltiger Abgasstrom erhalten wird,
g) Zuführen des Abgasstroms aus Schritt f) in einen Abgaskondensator und Entfernen des in dem Abgasstrom enthaltenen Toluols in diesem Abgaskondensator, wobei alle Abgasströme aus den Schritten a), b) und c) sowie optional d) und optional e) vereinigt und entweder in den dem Abgaskondensator zuzuführenden Abgasstrom oder in den den Abgaskondensator verlassenden Abgasstrom eingespeist werden,
wobei das Verfahren den weiteren Schritt umfasst:
h) Zuführen des in Schritt g) nach der Auskondensation des Toluols anfallenden Abgasstroms zu einer thermischen Abluftreinigung, wobei in den dem Abgaskondensator zuzuführenden Abgasstrom oder in den den Abgaskondensator verlassenden Abgasstrom Stickstoff zugegeben wird, wobei bevorzugt eine Stickstoffkonzentration im Abgasstrom von wenigstens 0,1 Vol.-% eingestellt wird, besonders bevorzugt von wenigstens 0,5 Vol.-%.

Dinitrotoluol (DNT) ist ein Zwischenprodukt für die Herstellung von Toluylendiisocyanat (TDI), das ein wichtiges, im großtechnischen Maßstab hergestelltes Vorprodukt zur Herstellung von Polyurethanen ist.

Die Herstellung des Dinitrotoluols durch Nitrierung von Toluol mit Nitriersäure (Gemisch von Salpetersäure und Schwefelsäure) war bereits Gegenstand zahlreicher Veröffentlichungen und Patentanmeldungen (Ullmanns Enzyklopedie der technischen Chemie, 4.Auflage, Band 17, Seite 391 ff, 1979, Verlag Chemie Weinheim / New York). Die industrielle Herstellung erfolgt, wie beispielsweise in H. Hermann, J. Gebauer, P. Konieczny, "Industrial Nitration of Toluene to Dinitrotoluene" in ACS-Symposium, Series 623, 234-249, 1996, ed. L.F.Albright, R.V.C Carr, R.J. Schmitt beschrieben, vorwiegend isotherm mit Salpetersäure in Gegenwart von Schwefelsäure als Katalysator kontinuierlich in zwei Stufen derart, dass
a) das in der Dinitrierung (Nitrierung von Mononitrotoluol - MNT - zu DNT) erhaltene Reaktionsgemisch durch Phasentrennung getrennt wird und die dabei erhaltene Abfallsäure mit Salpetersäure wieder aufkonzentriert wird und anschließend mit Toluol gemischt wird und der Mononitrierung (Nitrierung von Toluol zu MNT) zugeführt wird, und
b) das Reaktionsgemisch der Mononitrierung nach erfolgter Umsetzung in einer Separationsstufe in eine das Mononitrotoluol enthaltende organische und eine vorwiegend die Schwefelsäure enthaltende wässrige Phase ("Abfallsäure" / "Spent Acid") aufgetrennt wird, und
c) die in b) gewonnene, das Mononitrotoluol enthaltende organische Phase der Dinitrierung zugeführt und dort das Mononitrotoluol mit Salpetersäure in Gegenwart von Schwefelsäure zu Dinitrotoluol umgesetzt wird.

Üblicherweise wird zur Erzielung handelsüblicher Spezifikationen das so gewonnene Roh-DNT in nachgeschalteten Stufen, vorwiegend Wäschen, aufbereitet und so weitgehend von gelösten Schwefel- und Salpetersäuregehalten wie auch von in den Reaktionsstufen gebildeten Nebenkomponenten, z.B. Mono-, Di- und Trinitrokresole (im Folgenden unter dem Begriff Nitrokresole zusammengefasst), Pikrinsäure und Nitrobenzoesäuren, befreit. Typische handelsübliche DNT-Produkte weisen DNT-Gehalte > 98,5 Gew.-%, weniger als 0,1 Gew.-% Mononitrotoluol, weniger als 0,1 Gew.-% Trinitrotoluol und weniger als 0,1 Gew.-% sonstiger Nebenkomponenten, bezogen auf das Gewicht der DNT-Produkt Gemisches, bei DNT-Ausbeuten von > 98 % sowie Toluolumsätzen von > 99,9 % auf. Wesentlich ist ebenfalls das Gewichts-Verhältnis der 2,4- und 2,6-DNT-Isomeren in der Summe zu den 2,3-, 3,4-, 2,5 und 3,5-DNT-Isomeren in der Summe. Handelsüblich spezifikationsgerecht liegt der Gehalt der 2,4- und 2,6-DNT-Isomeren in der Summe im Roh-DNT bei > 95 Gew.-%, bezogen auf das Gewicht des Roh-DNT. Bevorzugt beträgt dabei der Gehalt des 2,4-DNT 79,0 - 81,0 Gew.-%, bezogen auf die Summe der Gewichte des 2,4-DNT und des 2,6-DNT. Entsprechend beträgt der Gehalt des 2,6-DNT 19,0 - 21,0 Gew.-%, bezogen auf die Summe der Gewichte des 2,4-DNT und des 2,6-DNT.

Neben dem Roh-DNT wird in dem Verfahren in der Auftrennung des in der Mononitrierung erhaltenen Reaktionsgemischs Abfallsäure gewonnen, die als zweiter Massenstrom das System verlässt. Die Abfallsäure weist üblicherweise einen Schwefelsäuregehalt von 70 - 80 Gew.-% auf und enthält üblicherweise > 0,1, bevorzugt > 0,2 bis 3,0 Gew.- % an nicht umgesetzter Salpetersäure, Nitrose aus in Nebenreaktionen ablaufenden Oxidationsvorgängen, > 0,2 Gew.-% an MNT, das in der Phasentrennung nicht abgeschieden worden ist, sowie üblicherweise Wasser in einem Konzentrationsbereich von > 16 bis < 30 Gew.-% (umfassend das mit der frisch in das Verfahren eingesetzten Schwefelsäure eingetragene, in der Salpetersäure enthaltene sowie das bei der Nitrierungen des Toluols und des Mononitrotoluols entstehende Wasser), jeweils bezogen auf das Gewicht der Abfallsäure.

In EP 1 496 043 A1 wird ein Verfahren zur Aufarbeitung von wässrigen Abwässern, die bei der Nitrierung von Toluol zu Dinitrotoluol mit Nitriersäure anfallen, beschrieben, wobei die sauren und alkalischen Abwässer aus der Wäsche von Dinitrotuluol und das wässrige Destillat aus der Schwefelsäureaufkonzentrierung vereinigt werden, so dass sich ein pH-Wert von unter 5 (gemessen bei 70 °C) einstellt. Anschließend werden die entstehende wässrige und organische Phase durch Phasentrennung getrennt. Die in der wässrigen Phase enthaltenen organischen Komponenten werden mit Toluol extrahiert und die mit den organischen Komponenten angereicherte Toluolphase wird dann der Nitrierung von Toluol zugeführt. Bei der beschriebenen Extraktion handelt es sich *um einen von der Wäsche des rohen Dinitrotuluols verschiedenen Schritt.* Die Anmeldung offenbart ferner, dass die wässrige Phase aus der Extraktion einer Dampfstrippung zugeführt werden kann. Das erhaltene Wasserdampf-Toluol-Gemisch wird kondensiert; das im Kondensat enthaltene Toluol kann nach Phasentrennung der Nitrierung zurückgeführt werden. Die Anmeldung offenbart nicht, Abgasströme aus den Gasräumen der in der Nitrier-Reaktion, der Wäsche und/oder der DNT-Wasser-Phasentrennung eingesetzten Apparate einer Kondensation zur Abtrennung von Toluol zu unterziehen. Die Anmeldung offenbart ebenfalls nicht, dass bei der Kondensation des Wasserdampf-Toluol-Gemisches ein Abgasstrom anfällt, der in Gegenwart von Stickstoff verbrannt wird.

Die in der Nitrierung entstehenden Stickoxide (NOₓ) können wie in US 5 313 009 beschrieben mittels Alkalilauge behandelt werden und als Natriumnitrat und -nitrit herausgewaschen werden. Zusätzlich wird auch noch Kohlendioxid, das im Nitrierprozess entsteht, als Natriumcarbonat gebunden.

In US 5 963 878 wird ein Verfahren offenbart, wobei von strategischen Bereichen des Nitriersystems NOₓ-Gase gewonnen, mit Luft und Wasser, beispielsweise in einer Einheit mit gepacktem Bett, bei höheren Temperaturen und unter Druck in Berührung gebracht werden, wobei die NOₓ-Gase durch das Wasser unter Bildung schwacher Salpetersäure absorbiert werden. Die schwache Salpetersäure wird in den Reaktionsprozess zurückgeführt. Kohlendioxid wird in einem NOₓ-Gaswaschturm nicht absorbiert, wenn der Gaswaschturm in einem sauren Modus betrieben wird.

Sauberes NOₓ-freies Abluftgas wird aus der Einheit mit gepacktem Bett als Schornsteinluft abgelassen.

All den Verfahren ist gemein, dass eine weitere Behandlung der Abgase von Nitrieranlagen nicht vorgesehen ist.

EP 2 719 682 A1 befasst sich mit einem Verfahren zur Herstellung von Nitrobenzol, bei dem die im Prozess anfallenden Abgasströme umfassend Benzol und (in Spuren) Nitrobenzol, ggf. Leicht- und Mittelsieder, ggf. nichtkondensierbare Gase sowie ggf. Wasser, optional nach Entfernung von Stickoxiden, in einer Absorptionskolonne mit Nitrobenzol, das nur sehr geringe Mengen (maximal 50 ppm) an Benzol enthält und das über einen Flüssigkeitsverteiler mit 50 bis 200 Tropfsteilen pro Quadratmeter verteilt wird, gewaschen werden, wobei (i) ein flüssiger Strom umfassend Benzol und Nitrobenzol, ggf. organische Leicht- und Mittelsieder und (ii) an Benzol und ggf. organischen Leicht- und Mittelsiedern abgereichertes Abgas erhalten werden. Dieses Abgas eignet sich besonders für die Verbrennung in einer thermischen Abluftreinigung.

WO 2014/016290 A1 befasst sich mit einem kontinuierlichen adiabaten Verfahren zur Herstellung von Nitrobenzol durch Nitrierung von Benzol mit Mischungen aus Schwefel- und Salpetersäure unter Einsatz eines stöchiometrischen Überschusses an Benzol, bei dem der Gehalt an organischen Verbindungen in der Kreislaufschwefelsäure mindestens während der Anfahrzeit der Produktionsanlage stets kleiner als 1,0 Massen-%, bezogen auf die Gesamtmasse der Kreislaufschwefelsäure, gehalten wird. Dies wird bevorzugt erreicht, indem entweder nach Beendigung oder vor Beginn eines Produktionszyklus die Kreislaufschwefelsäure bei erhöhter Temperatur im Kreis gefahren wird, und so im Verdampfungsapparat für die Aufkonzentrierung der Schwefelsäure die in der Schwefelsäure enthaltenen Organika, bevorzugt umfassend Nitrobenzol und Spuren an Benzol, Dinitrobenzol und Nitrophenole, abgetrennt werden.

EP 0 155 586 A1 offenbart ein Verfahren zur Herstellung von Dinitrotoluol durch zweistufige Umsetzung von Toluol mit Salpetersäure in Gegenwart von Schwefelsäure, wobei in der ersten Stufe Toluol zu Mononitrotoluol nitriert wird unter Verwendung von Absäure aus der zweiten Stufe, in der das Mononitrotoluol zu Dinitrotoluol nitriert wird unter Verwendung von aufkonzentrierter Absäure aus der ersten Stufe, wobei die Absäure in indirekt beheizten Eindampfern unter Vakuum aufkonzentriert wird und Mononitrotoluol in die überhitzten Brüden des Eindampfers gegeben wird. Dabei kann die Absäure zur Entfernung von NOₓ vor der Eindampfung mit Dampf gestrippt werden, wobei die nitrosen Gase nach Kondensation des Wasserdampfes und der Wasserdampf-flüchtigen Verbindungen in Natronlauge unter Bildung von Natriumnitrit absorbiert und einer Weiterverwendung zugeführt werden können. Alternativ können die nitrosen Gase in einer reduzierenden Flamme zerstört werden.

WO 2011/082977 A1 befasst sich mit einem Verfahren zur Aufarbeitung von alkalischen Prozessabwässern aus der Nitrierung von aromatischen Verbindungen zu Mono-, Di- und Trinitroaromaten, das es ermöglichen soll, die anfallenden Neben- und Abfallprodukte der Nitrierung im Verfahren wiederverwerten zu können und/oder mit einer geringeren Belastung an unerwünschten Bestandteilen der Abwasserentsorgung bzw. -aufarbeitung zuführen können. Zu diesem Zweck wird vorgeschlagen, das anfallende Prozessabwasser durch Zugabe von Säure auf einen pH-Wert unterhalb von 5 anzusäuern und die sich dabei ausbildende gasförmige, NOₓ-haltige Phase abzuführen. Diese NOₓ-haltige Phase kann entweder zu Salpetersäure weiterverarbeitet werden, und zwar insbesondere ohne vorherige Reinigung und Abtrennung von anderen Gasbestandteilen direkt der Absorptionskolonne der Salpetersäureherstellung zugeführt werden, oder sie kann verbrannt werden.

EP 1 880 989 A1 befasst sich mit der Aufgabe, ein Verfahren zur Umsetzung von Toluol mit Salpetersäure zu Dinitrotoluol in Gegenwart von Schwefelsäure zur Verfügung zu stellen, das bei einem einfachen Anlagenauftbau einen hohen Toluolumsatz mit hoher Selektivität bezüglich DNT sowie eine hohe und selektive Nutzung der direkt in die Reaktionsstufen eingesetzten Salpetersäure gestattet. Zu diesem Zweck wird ein Verfahren zur Herstellung von Dinitrotoluol in zwei Stufen vorgeschlagen, bei dem in der Mononitrierstufe das Gewichts-Verhältnis der wässrigen zur organischen Phase auf Werte > 2 : 1 und in der Dinitrierstufe auf Werte > 1 ,5 : 1 eingestellt wird und in beiden Schritten jeweils die organische Phase als disperse Phase in der wässrigen Phase (homogene Phase) dispergiert wird und gleichzeitig insgesamt weniger als 2,06 mol Salpetersäure pro mol Toluol in das Verfahren eingespeist werden. In EP1880989A1 wird beschrieben, dass zahlreiche Untersuchungen in der Vergangenheit darauf abzielten die Qualität des Roh-DNT zu verbessern und somit die Ausbeute bezüglich Toluol und Salpetersäure zu erhöhen. Bei allen Verfahren zur Herstellung von DNT durch Nitrierung von Toluol mit Salpetersäure ist für eine wirtschaftliche Verfahrensführung Voraussetzung, dass die bei dem Verfahren anfallenden Absäuren so wieder aufbereitet werden können, dass sie erneut als Reaktionsmedium in den Reaktionsprozess eingesetzt werden können (wie beispielsweise in EP 155 586 A und US 5 275 701 A beschrieben).

Wesentlich für die Auswahl eines DNT-Nitrierverfahrens sind jedoch zusätzlich auch seine inhärente Sicherheit, die Robustheit mit der es betrieben werden kann, die Selektivität und Vollständigkeit, mit der das Toluol zu Dinitrotoluol umgesetzt werden kann, sowie der spezifische Einsatz an Salpetersäure, der für die Umsetzung des Toluols zu Dinitrotoluol erforderlich ist. Dank dieser Entwicklungen sind die heutigen DNT-Verfahren so ausgereift, dass es allen gelingt, ein DNT mit hohen Ausbeuten herzustellen das einen geringen Gehalt an Nebenprodukten aufweist.

Beim Betreiben einer Nitrieranlage zur Herstellung von Dinitrotoluol aus Toluol wurde allerdings gefunden, dass die isolierte molare Menge an Endprodukt Dinitrotoluol und den dem Verfahren inhärent dazugehörenden Nebenprodukten wie Kresolen und deren Abbauprodukten sowie Mononitrotoluol und Trinitrotoluol geringer war als die entsprechende Menge an eingesetztem Toluol. Der Auslass für diesen Verlust ist offensichtlich das Abgas der Produktionsanlage, was aus ökologischer Sicht unerwünscht ist. Darüber hinaus ist der Verlust auch mit ökonomischen Nachteilen verbunden.

Es bestand daher ein Bedarf an einer Verbesserung der bestehenden Nitriertechnik dahingehend, dass die Umweltbelastung mit Organika über das Abgas verringert wird. Erstrebenswert war darüber hinaus, die Verringerung der Umweltbelastung möglichst so durchzuführen, dass sie mit wirtschaftlichen Vorteilen verbunden ist. Insbesondere war erstrebenswert, im Abgas mitgerissenes Toluol einer wirtschaftlichen Verwertung zuzuführen.

Die Aufgabe wird gelöst durch ein Verfahren zur Herstellung von Dinitrotoluol, umfassend die Schritte:
a) Nitrierung von Toluol mit einem Gemisch aus Salpetersäure und Schwefelsäure mit anschließender Abtrennung einer bei der Nitrierung entstehenden schwefelsäurehaltigen wässrigen Phase, wobei ein Roh-Dinitrotoluol erhalten wird,
b) Waschen des Roh-Dinitrotoluols in einer Wasserwäsche mit neutralem und/oder alkalischem Waschwasser, wobei nach Abtrennung des in der letzten Wäsche eingesetzten Waschwassers ein vorgereinigtes Dinitrotoluol erhalten wird, welches neben Dinitrotoluol zumindest Wasser enthält und
c) Abtrennen des Wassers aus dem vorgereinigten Dinitrotoluol,
d) Sammeln der Abwässer aus den Schritten a), b) und/oder c),
e) optional, Extraktion der gesammelten Abwässer aus Schritt d) mit Toluol und Rückführung der so erhaltenen organischen Phase in Schritt a),
f) Befreiung der gesammelten Abwässer aus Schritt d), bzw., falls der optionale Schritt e) durchgeführt wird, des extrahierten Abwassers aus Schritt e), von Toluol in einem Toluolstripper, wobei ein Toluol-haltiger Abgasstrom erhalten wird,
g) Zuführen des Abgasstroms aus Schritt f) in einen Abgaskondensator und Entfernen des in dem Abgasstrom enthaltenen Toluols in diesem Abgaskondensator, wobei alle Abgasströme aus den Schritten a), b) und c) sowie optional d) und optional e) vereinigt und entweder in den dem Abgaskondensator zuzuführenden Abgasstrom oder in den den Abgaskondensator verlassenden Abgasstrom eingespeist werden,
wobei das Verfahren den weiteren Schritt umfasst:
h) Zuführen des in Schritt g) nach der Auskondensation des Toluols anfallenden Abgasstroms zu einer thermischen Abluftreinigung, wobei in den dem Abgaskondensator zuzuführenden Abgasstrom oder in den den Abgaskondensator verlassenden Abgasstrom Stickstoff zugegeben wird, wobei bevorzugt eine Stickstoffkonzentration im Abgasstrom von wenigstens 0,1 Vol.-% eingestellt wird, besonders bevorzugt von wenigstens 0,5 Vol.-%.

Der vorliegenden Erfindung liegt die Erkenntnis zugrunde, dass durch Zuführen des Abgasstroms aus Schritt f) und gegebenenfalls der vereinigten Abgasströme aus den Schritten a), b), und c), sowie optional d) und optional e) und Auskondensieren der organischen gasförmigen Bestandteile nicht nur der Anteil an organischen Verbindungen im Abgasstrom reduziert werden kann, sondern dass Toluol den Großteil der darin enthaltenen Verbindungen ausmacht, welches nach dem Auskondensieren und Aufreinigen dem Prozess wieder zugeführt oder aber auch verbrannt werden kann. Dabei wird erfindungsgemäß unter einem *"Abgasstrom"* ein solcher Gasstrom verstanden, der in einem der erfindungsgemäßen Schritte aus dem Gasraum oberhalb des flüssigen Verfahrensprodukts des jeweiligen Verfahrensschrittes abgeführt wird oder der als gasförmiges Verfahrensprodukt anfällt. Bspw. findet die Nitrierung in Schritt a) in einem Reaktor statt, in dem sich eine flüssige Phase befindet, in welcher die chemische Umsetzung stattfindet. Über dieser flüssigen Phase befindet sich, da die flüssige Phase das Innenvolumen des Reaktors nicht vollständig ausfüllt, ein Gasraum. Ebenso sind in den Vorrichtungen der Schritte b) (z. B. gerührte Waschbehälter), c) (z. B. Phasentrennapparate) und d) (z. B. Lagertanks) jeweils flüssige Phasen mit darüber liegenden Gasräumen vorhanden. In Schritt f) fällt ein mit Toluol beladenes Strippgas an, das erfindungsgemäß als Abgasstrom bezeichnet wird.

Das *Entfernen von Toluol* in Schritt g) geschieht erfindungsgemäß möglichst vollständig. Bevorzugt wird ein Restgehalt an Toluol in dem den Abgaskondensator verlassenden Abgasstrom eingestellt, welcher maximal 10 % der in dem dem Abgaskondensator zugeführten Abgasstrom vorhandenen Toluolmenge entspricht.

Durch die erfindungsgemäße Vorgehensweise wird außerdem die Verfahrens- und Anlagensicherheit im Umgang mit Stickoxid haltigen Toluolabgasen gewährleistet. Das Verfahren wird vorzugsweise kontinuierlich betrieben.

Das Wort "ein" ist im Rahmen dieser Erfindung im Zusammenhang mit zählbaren Größen nur dann als Zahlwort und nicht lediglich als unbestimmter Artikel zu verstehen, wenn dies ausdrücklich gesagt wird (z. B. durch den Ausdruck "genau ein"). Bspw. schließt der Ausdruck "ein Kondensator" die Möglichkeit des Vorhandenseins mehrerer (in Reihe oder parallel geschalteter) Kondensatoren nicht aus.

Als *nicht kondensierbare Gase* werden im Rahmen der vorliegenden Erfindungen solche Stoffe verstanden, die unter Normalbedingungen gasförmig vorliegen und mit großtechnisch üblichen Kondensatoren (Temperaturen bis -20 °C) nicht verflüssigt werden können. Typische Beispiele sind Stickoxide (NOₓ) und Kohlendioxid.

Die Zugabe von Stickstoff im erfindungsgemäßen Schritt h) bewirkt, dass das in dem Abgasstrom enthaltende Gemisch aus Stickoxiden und Toluol bei der im Abgasstrom vorherrschenden Temperatur und dem vorherrschenden Druck außerhalb des Konzentrationsbereiches zur Bildung eines explosionsfähigen Gemisches liegt. Bevorzugt ist die Zugabe von Stickstoff in den den Abgaskondensator verlassenden Abgasstrom, welcher der thermischen Abluftreinigung zugeführt wird, denn vor der Kondensation enthält der Abgasstrom i. d. R. noch Wasser, was der Bildung eines explosionsfähigen Gemisches entgegenwirkt. Stickstoff wird dem Abgasstrom bevorzugt unmittelbar nach Verlassen des Kondensators zugegeben.

Bei dem erfindungsgemäßen Verfahren wird im Schritt g) der Abgasstrom aus Schritt f) dem Abgaskondensator zugeführt, wobei alle Abgasströme aus den Schritten a), b) und c) sowie optional d) und optional e) vereinigt und entweder in den dem Abgaskondensator zuzuführenden Abgasstrom aus Schritt f) oder - bevorzugt - in den den Abgaskondensator verlassenden Abgasstrom eingespeist werden.

Die Nitrierung von Toluol kann beispielsweise in an sich bekannter Weise in einem kontinuierlichen, zweistufigen Verfahren durchgeführt wird, wobei in der ersten Stufe ein Roh-Mononitrotoluol erhalten wird, welches in der zweiten Stufe zu dem Roh-Dinitrotoluol umgesetzt wird.

Das Waschen des Roh-Dinitrotoluols kann in der Reihenfolge neutrales, dann alkalisches und zuletzt neutrales Waschwasser erfolgen. Die Abtrennung in Schritt c) kann beispielsweise in einem Lagertank mit Scheidevorrichtung vollzogen werden.

Nach einer bevorzugten Ausführungsform des erfindungsgemäßen Verfahrens wird im Abgaskondensator in Schritt g) ein absoluter Druck im Bereich von 10 mbar bis 1200 mbar eingestellt, bevorzugt von 100 mbar bis 1150 mbar. Unabhängig hiervon kann im Abgaskondensator in Schritt g) eine Temperatur von 20 °C bis 75 °C eingestellt werden, bevorzugt von 25 °C bis 60 °C, und besonders bevorzugt von 30 °C bis 45 °C. Als Abgaskondensator kann beispielweise ein Wärmetauscher verwendet werden. Die Temperatur des Abgases wird dabei bevorzugt in der zuführenden Rohrleitung unmittelbar vor dem Abgaskondensator gemessen. Die Temperatur des Abgases nach der Kondensation beträgt beispielsweise 10 °C bis 30 °C, bevorzugt 15 °C bis 25 °C.

Als Abgaskondensator können im Rahmen des erfindungsgemäßen Verfahrens beispielsweise Kondensatoren mit Kühlschlange oder Wendelrohr, Doppelrohrkühler sowie Rohrbündelwärmetauscher eingesetzt werden. Geeignete Materialien zur Konstruktion eines Abluftkondensators sind zum Beispiel Glas und Metalle wie Stahl, insbesondere korrosionsbeständiger legierter Stahl oder emaillierter Stahl. Glas bietet den Vorteil, dass eventuell auftretende Ablagerungen auf der Produktseite des Kondensators leicht erkannt werden können, eine Ausführung in Stahl liefert hingegen mehr Freiheitsgrade bezüglich des Druckes. Als Kühlmedium dient vorzugsweise ein Kühlwasserstrom geeigneter Temperatur. Andere Wärmeträgermedien wie zum Beispiel Wärmeträgeröle oder organische Lösungsmittel können jedoch ebenso genutzt werden. Das im Abluftkondensator aus dem Abgasstrom verflüssigte Gemisch, im Wesentlichen bestehend aus Wasser, Toluol und gegebenenfalls gelösten Stickoxiden, wird als Ablauf gesammelt und seiner weiteren Verarbeitung zugeführt.

In vorteilhafter Ausgestaltung des erfindungsgemäßen Verfahrens kann das in Schritt g) auskondensierte Toluol der Nitrierung in Schritt a) zugeführt werden. Vor der Zuführung zur Nitrierung kann das Toluol, falls gewünscht, einer Aufreinigung unterzogen werden. Bevorzugt wird das Toluol in die Mononitrierungsstufe zurückgeführt und zwar bevorzugt in die Abwasseraufarbeitung der Mononitrierung.

Bei dem erfindungsgemäßen Verfahren kann der dem Abgaskondensator zuzuführende oder - bevorzugt - der den Abgaskondensator verlassende Abgasstrom von Stickoxiden befreit werden. Die Abtrennung von Stickoxiden erfolgt besonders bevorzugt nach der Zugabe von Stickstoff in den den Abgaskondensator verlassenden Gasstrom. Die Stickoxid-Abtrennung kann beispielweise durch Einsatz eines NOₓ-Absorbers erfolgen, wie in US 5,963,878 beschrieben, insbesondere in Spalte 2, Z. 12 bis Spalte 3, Z. 27.

Der in Schritt g) nach der Auskondensation des Toluols anfallende Abgasstrom wird anschließend einer thermischen Abluftreinigung zugeführt, beispielsweise einer Verbrennungseinrichtung. Dabei kann der Abgasstrom nach Durchlaufen des Abgaskondensators vor dem Zuführen zur thermischen Abluftreinigung mit NOₓ-haltigen Abgasen aus dem Nitrierprozess vereinigt werden. Eine ggf. vorhandende Stickoxid-Abtrennung erfolgt dann nach Zugabe dieser NOₓ-haltigen Abgase. Alternativ kann das Stickoxid enthaltende Abgas, wie dem Fachmann bekannt, in eine für die Zusammensetzung geeignete thermische Abluftreinigung geführt werden.

Im Rahmen des erfindungsgemäßen Verfahrens kann weiter vorgesehen sein, dass die Schwefelsäure haltige wässrige Phase nach der Abtrennung gereinigt sowie, sofern gewünscht, aufkonzentriert und/oder rezykliert wird.

Im erfindungsgemäßen Verfahren werden in Schritt d) die Abwässer aus der Nitrierung, der Wäsche und/oder der Wasserabtrennung aus dem vorgereinigten Dinitrotoluol bzw. der in der ggf. durchgeführten Extraktion erhaltenen wässrigen Phase, das heißt die Abwässer aus den Schritten a), b) und/oder c) gesammelt. Da die Abwässer aus der Schwefelsäureaufkonzentration (sulfuric **a**cid concentration) SAC ebenfalls Toluol enthalten können, ist es möglich, auch diese Abwässer in Schritt d) mit den übrigen Abwässern zu vereinigen. Die gesammelten Abwässer aus Schritt d) bzw., falls der optionale Schritt e) durchgeführt wird, des extrahierten Abwassers aus Schritt e), werden anschließend in einem Toluolstripper durch Dampfstrippung von Toluol befreit (Schritt f)). In bevorzugter Ausgestaltung der Erfindung werden dann nur die feuchten Abgase aus dem Toluolstripper in einem Kondensator, wie einem Rohrbündelwärmeaustauscher, von Toluol und Wasser befreit (Schritt g)). Dann wird diesem im Wesentlichen Organika-freien Abgas Stickstoff zugeführt (Schritt h)). Dieser Abgasstrom wird anschließend mit den Abgasströmen aus den Schritten a), b) und c) sowie optional d) und optional e) vereinigt und vorzugsweise zusammen mit im Prozess angefallenen NOₓ-Gasen verbrannt.

Nachstehend wird die Erfindung im Detail erläutert. Verschiedene Ausführungsformen sind dabei beliebig miteinander kombinierbar, solange sich für den Fachmann aus dem Kontext nicht eindeutig das Gegenteil ergibt.

**Schritt a)** des erfindungsgemäßen Verfahrens kann im Prinzip nach allen im Stand der Technik bekannten Verfahren zur Nitrierung von Toluol erfolgen. Bevorzugt ist die Umsetzung von Toluol mit einem Gemisch aus Salpetersäure und Schwefelsäure unter kontinuierlicher, isothermer, zweistufiger Verfahrensführung, wie sie in EP 1 880 989 A1 sowie darin zitierten Schriften beschrieben ist, was hiermit als Bestandteil der vorliegenden Offenbarung gilt. Die Reinigung und Aufkonzentrierung (SAC) der anfallenden Schwefelsäure zur Wiederverwertung im Nitrierprozess kann nach den gängigen, dem Stand der Technik entsprechenden Verfahren durchgeführt werden. Ein bevorzugtes Verfahren hierfür ist in DE 196 36 191 B4 beschrieben, was hiermit als Bestandteil der vorliegenden Offenbarung gilt. Hier sind insbesondere die vor den Aufkonzentrierstufen anfallenden Abgasströme geeignet für eine Gewinnung und Verarbeitung von Stickoxiden zu Salpetersäure gemäß US 5 963 878.

In **Schritt b)** des erfindungsgemäßen Verfahrens können die einzelnen Waschschritte prinzipiell in beliebiger Reihenfolge durchgeführt werden. Bevorzugt ist jedoch die Reihenfolge (1) neutrale Wäsche - (2) alkalische Wäsche - (3) neutrale Wäsche. (Das im ersten Waschschritt nach Phasentrennung erhaltene Abwasser ist infolge seines Gehalts an ausgewaschener Säure sauer. Daher wird diese erste Wäsche gelegentlich auch als "saure Wäsche" bezeichnet.) Es sind jedoch alle weiteren denkbaren Kombinationen möglich, ebenso eine Waschfolge ohne alkalische Wäsche. Die dabei anfallenden Abwasserströme der neutralen und der alkalischen DNT-Wäsche und bevorzugt auch der Schwefelsäureaufkonzentrierung werden bevorzugt vereint **(Schritt d)**). Nach der Zusammenführung der genannten Ströme scheidet sich eine organische Phase ab. Diese organische Phase besteht aus MNT und DNT sowie Nebenprodukten der Nitrierung, vorwiegend Nitrokresolen, Pikrinsäure und Nitrobenzoesäuren. Zur Abtrennung der gebildeten organischen Phase werden die vereinigten Abwasserströme anschließend in ein geeignetes Scheidegefäß geleitet. Besonders bevorzugt erfolgt Schritt b) nach der in EP 1 496 043 A1 beschriebenen Vorgehensweise, welche hiermit als Bestandteil der vorliegenden Offenbarung gilt. In den Apparaten des Schrittes b) können im sauren und neutralen Milieu NOₓ haltige Abluft anfallen.

Das gereinigte, feuchte DNT wird in einem Lagertank zwischengelagert, wobei sich in **Schritt c)** Wasser absetzt, was mittels einer Scheidevorrichtung entfernt wird. Bevorzugt wird dieses abgetrennte Wasser in Schritt d) mit den zuvor genannten wässrigen Strömen vereint.

In den bisher geschilderten Verfahrensschritten a) bis d) fallen gasförmige Abgasströme an, die aus den Gasräumen über dem flüssigen Verfahrensprodukt des jeweiligen Verfahrensschrittes abgeführt werden. Wird der jeweilige Verfahrensschritt a), b), c) oder d) unter Luftausschluss durchgeführt, wie es im erfindungsgemäßen Verfahren für alle diese Schritte bevorzugt ist, umfasst der Gasraum das jeweils zur Inertisierung eingesetzte Inertgas (bevorzugt Stickstoff). Anderenfalls umfasst der Gasraum Luft. In jedem Fall enthält der Gasraum auch Bestandteile des jeweiligen flüssigen Verfahrensprodukts. Dies ist darauf zurückzuführen, dass aus den flüssigen Verfahrensprodukten der einzelnen Verfahrensschritte nicht kondensierbare Gase infolge begrenzter Gaslöslichkeit in der flüssigen Phase aus dieser flüssigen Phase in den Gasraum ausgasen. Außerdem gehen organische Verbindungen je nach Dampfdruck anteilig in die Gasphase über. Die einzelnen Abgasströme enthalten in variierender Zusammensetzung nicht kondensierbare Gase (Kohlenmonoxid, Kohlendioxid, Sauerstoff, Stickoxide, Stickstoff), Toluol, Mononitrotoluol, Dinitrotoluol sowie ggf. Wasser. Verfahrensabgase werden bevorzugt von Apparaten in Schritt a) im Bereich der Nitrierung von Toluol zu Dinitrotoluol, der Phasentrennung, der Schwefelsäurereinigung und -aufkonzentrierung, von Apparaten und Behältern in Schritt b), der DNT-Wäsche und Abwasseraufbereitung, sowie von Behältern und Tanks aus Schritt c), der Lagerung, und Schritt d), der Abwassersammlung, entnommen und der Abgaskondensation in Schritt g) oder bevorzugt dem die Abgaskondensation verlassenden Abgasstrom zugeführt. Optional kann die Reinigung des Abgasstroms/der Abgasströme von Stickoxiden nach allen aus dem Stand der Technik bekannten Verfahren erfolgen. Bevorzugt ist dann der Einsatz eines NOₓ-Absorbers wie in US 5,963,878, insbesondere in Spalte 2, Z. 12 bis Spalte 3, Z. 27, beschrieben. Diese Stickoxidabtrennung erfolgt in einem Schritt g) nachgelagerten Schritt g.1). Dies ist besonders bevorzugt, um die Explosionsgrenzen des Abgasgemisches Toluol / Sauerstoff, die beim Einleiten von Sauerstoff in den Abgasstrom zur NOₓ-Absorption entstehen würden, zu vermeiden.

Die optionale Extraktion der gesammelten Abwässer aus **Schritt e)** mit Toluol erfolgt bevorzugt wie in EP 1 496 043 A1 beschrieben.

In **Schritt g)** des erfindungsgemäßen Verfahrens wird mindestens der Abgasstrom aus Schritt f) in einem Abgaskondensator behandelt, um mitgerissenes Toluol zurückzugewinnen.

Bevorzugt wird der Druck in dem in Schritt g) eingesetzten Abgaskondensator so gewählt, dass sich Toluol und Wasser optimal auskondensieren lassen. Die Erfindung betrifft demnach insbesondere auch ein Verfahren, bei dem Schritt g) bei einem absoluten Druck von 10 mbar bis 1200 mbar, bevorzugt von 100 mbar bis 1150 mbar, besonders bevorzugt von 100 mbar bis 500 mbar betrieben wird. Dabei wird der Druck bevorzugt im Eingang des Abgaskondensators (Wärmeaustauscher) gemessen.

Bevorzugt ist ein Verfahren, bei dem das Abgas in Schritt g) in einem Abgaskondensator, der als Wärmeaustauscher betrieben wird, von Toluol und teilweise von Wasser durch Auskondensieren befreit, die org. Phase wiederum von Wasser befreit und das organische Kondensat einer geeigneten Aufarbeitung zugeführt wird, zum Beispiel der Abwasseraufarbeitung in Schritt b) oder der Mononitrierung von Toluol in Schritt a).

Geeignete Apparate werden bspw. beschrieben in Reinhard Billet; "Verdampfung und ihre technischen Anwendungen"; Verlag Chemie Weinheim - Deerfield Beach, Florida - Basel; 1981, Kapitel 4.1.2, Seite 208 bis 230.

Eine bevorzugte Ausführungsform zum Betreiben eines Abgaskondensators wird im Folgenden beschrieben:
Das Abgas des Toluolstrippers aus Schritt f) wird in einem Wärmeaustauscher kondensiert und von Toluol befreit (Schritt g)). Anschließend wird das so behandelte Abgas mit Stickstoff versetzt und mit den anderen nicht Toluol haltigen Abgasen aus der SAC, das optional nach einem in der Literatur gängigen Verfahren von NOₓ befreit wird, der DNT-Reaktion und den DNT-Tanks vereinigt und in einer thermischen Abluftreinigung ("TAREX") verbrannt (Schritt h)).

Die vorliegende Erfindung ist im Folgenden anhand von Ausführungsbeispielen näher erläutert.

### Beispiele

### Beispiel 1 (Versleichsbeispiel): unbehandeltes Abgas (kein Toluolkondensator)

Gemäß den Schritten a) bis b) des erfindungsgemäßen Verfahrens wird zunächst bei einer Produktionslast von 26 Tonnen pro Stunde feuchtes, reines DNT hergestellt und dieses in Schritt c) in einem Lagertank mittels Scheidevorrichtung von Wasser befreit. Es verbleibt reines feuchtes DNT. Die aus Schritt b) verbliebenen 69 t/h an Waschwasser werden in eine Strippkolonne geführt und mit 3,5 t/h 6 bar Dampf beaufschlagt. Der Sumpfaustrag der Strippkolonne besteht aus warmen Wasser und ca. 0,5 kg/h Toluol, die zur biologischen Aufarbeitung abgeführt werden. Die Brüden am Kopf der Stripperkolonne bei Atmosphärendruck enthalten neben Wasser 75 kg/h Toluol, die mit dem Abgas verbrannt werden.

Die Analytik des Abgasstromes erfolgte mittels Gaschromatographie.

### Beispiel 2 (Vergleichsbeispiel): Rückgewinnung von Toluol aus dem Abgas ohne Zugabe von Stickstoff

Gemäß den Schritten a) bis b) des erfindungsgemäßen Verfahrens wird zunächst bei einer Produktionslast von 26 Tonnen pro Stunde feuchtes, reines DNT hergestellt und dieses in Schritt c) in einem Lagertank mittels Scheidevorrichtung von Wasser befreit. Es verbleibt reines feuchtes DNT. Die aus Schritt b) verbliebenen 69 t, 61 °C wannen, Waschwassers werden in eine Strippkolonne geführt und mit 3,5 t 3,5 bar Dampf beaufschlagt (Dampfstrippung, Schritt f)). Der Sumpfaustrag der Strippkolonne besteht aus 69,4 t, 67 °C wannen Wasser, der zur biologischen Aufarbeitung abgeführt wird. Es fallen 3 t/h Brüden, die 2,91 t Wasser und 81 kg/h Toluol enthalten, bei einer Temperatur von 65 °C am Kopf der Stripperkolonne bei einem Druck von 250 mbar an. Diese Brüden werden der Abgaskondensation in Schritt g) zugeführt. In einem ersten Kondensator werden 2,9 t/h an Wasser und 1,9 kg/h an Toluol kondensiert. Der verbliebene dampfförmige Strom von 79,2 kg/h Toluol und 14 kg/h Wasser wird dann über einen Dampfstrahler in eine zweite Kondensationsstufe bei einem bar Druck geleitet, wobei 75 kg/h Toluol und restliches Wasser bei 19 °C kondensieren. Der verbliebene Gasstrom mit einer restlichen Toluolmenge von 4 kg/h wird in einer thermischen Abluftreinigung verbrannt.

Die Analytik des Abgasstromes erfolgte mittels Gaschromatographie.

### Beispiel 3 (erfindungsgemäßes Beispiel): Rückgewinnung von Toluol aus dem Abgas und sicheres Betreiben der Abgasleitungen

Gemäß den Schritten a) bis b) des erfindungsgemäßen Verfahrens wird zunächst bei einer Produktionslast von 26 Tonnen pro Stunde feuchtes, reines DNT hergestellt und dieses in Schritt c) in einem Lagertank mittels Scheidevorrichtung von Wasser befreit. Es verbleibt reines feuchtes DNT. Die aus Schritt b) verbliebenen 69 t, 61 °C warmen, Waschwassers werden in eine Strippkolonne geführt und mit 3,5 t 3,5 bar Dampf beaufschlagt (Dampfstrippung, Schritt f)). Der Sumpfaustrag der Strippkolonne besteht aus 69,4 t, 67 °C warmen Wasser, der zur biologischen Aufarbeitung abgeführt wird. Es fallen 3 t/h Brüden, die 2,91 t Wasser und 81 kg/h Toluol enthalten, bei einer Temperatur von 65 °C am Kopf der Stripperkolonne bei einem Druck von 250 mbar an. Diese Brüden werden der Abgaskondensation in Schritt g) zugeführt. In einem ersten Kondensator werden 2,9 t/h an Wasser und 1,9 kg/h an Toluol kondensiert. Der verbliebene dampfförmige Strom von 79,2 kg/h Toluol und 14 kg/h Wasser wird dann über einen Dampfstrahler in eine zweite Kondensationsstufe bei einem bar Druck geleitet, wobei 75 kg/h Toluol und restliches Wasser bei 19 °C kondensieren. Direkt im Ausgang der zweiten Kondensationsstufe wird so viel Stickstoff in die der thermischen Abluftreinigung zuzuführenden Abgasleitung eindosiert, dass sich durch diese Zugabe von Stickstoff eine Stickstoffkonzentration im Abgasstrom von wenigstens 0,6 Vol.-% einstellt (Schritt h)). Der so explosionssicher gemachte Gasstrom mit einer restlichen Toluolmenge von 4 kg/h wird in einer thermischen Abluftreinigung verbrannt, wobei ein besonders sicheres Betreiben der Abgasleitung, die den Ausgang der zweiten Kondensationsstufe mit der thermischen Abluftreinigung verbindet, gewährleistet ist. Die Abgasströme aus den Schritten a), b) und c) werden vereinigt und enthalten < 5 ppm Toluol. Die vereinigten Abgasströme werden unmittelbar vor der thermischen Abluftreinigung in die Abgasleitung, die die zweite Kondensationsstufe mit der thermischen Abluftreinigung verbindet, eingespeist.

Die Analytik des Abgasstromes erfolgte mittels Gaschromatographie.

### Beispiel 4 (erfindungsgemäßes Beispiel): Weiterverwertung des aus dem Abgas gewonnenen Toluol

Das aus Beispiel 3 in der zweiten Kondensationsstufe angefallene Toluol/Wasser-Gemisch, das 75 kg/h an Toluol enthält, wird in einen Dekanter gefahren. Die organische Phase wird mit Frischtoluol vereinigt und zur Nitrierung geleitet und die wässrige Phase wird zur Abwasserstrippkolonne geführt.

## Patentansprüche

1. Verfahren zur Herstellung von Dinitrotoluol, umfassend die Schritte:
a) Nitrierung von Toluol mit einem Gemisch aus Salpetersäure und Schwefelsäure mit anschließender Abtrennung einer bei der Nitrierung entstehenden Schwefelsäure-haltigen wässrigen Phase, wobei ein Roh-Dinitrotoluol erhalten wird,
b) Waschen des Roh-Dinitrotoluols in einer Wasserwäsche mit neutralem und/oder alkalischem Waschwasser, wobei nach Abtrennung des in der letzten Wäsche eingesetzten Waschwassers ein vorgereinigtes Dinitrotoluol erhalten wird, welches neben Dinitrotoluol zumindest Wasser enthält und
c) Abtrennen des Wassers aus dem vorgereinigten Dinitrotoluol,
d) Sammeln der Abwässer aus den Schritten a), b) und/oder c),
e) optional, Extraktion der gesammelten Abwässer aus Schritt d) mit Toluol und Rückführung der so erhaltenen organischen Phase in Schritt a),
f) Befreiung der gesammelten Abwässer aus Schritt d), bzw., falls der optionale Schritt e) durchgeführt wird, des extrahierten Abwassers aus Schritt e), von Toluol in einem Toluolstripper, wobei ein Toluol-haltiger Abgasstrom erhalten wird,
g) Zuführen des Abgasstroms aus Schritt f) in einen Abgaskondensator und Entfernen des in dem Abgasstrom enthaltenen Toluols in diesem Abgaskondensator, wobei alle Abgasströme aus den Schritten a), b) und c) sowie optional d) und optional e) vereinigt und entweder in den dem Abgaskondensator zuzuführenden Abgasstrom oder in den den Abgaskondensator verlassenden Abgasstrom eingespeist werden,
**dadurch gekennzeichnet, dass** das Verfahren den weiteren Schritt umfasst:
h) Zuführen des in Schritt g) nach der Auskondensation des Toluols anfallenden Abgasstroms zu einer thermischen Abluftreinigung, wobei in den dem Abgaskondensator zuzuführenden Abgasstrom oder in den den Abgaskondensator verlassenden Abgasstrom Stickstoff zugegeben wird, wobei bevorzugt eine Stickstoffkonzentration im Abgasstrom von wenigstens 0,1 Vol.-% eingestellt wird, besonders bevorzugt von wenigstens 0,5 Vol.-%.

2. Verfahren nach einem der vorstehenden Ansprüche, **dadurch gekennzeichnet, dass** die Nitrierung von Toluol in Schritt a) in einem kontinuierlichen, zweistufigen Verfahren durchgeführt wird, wobei in der ersten Stufe ein Roh-Mononitrotoluol erhalten wird, welches in der zweiten Stufe zu dem Roh-Dinitrotoluol umgesetzt wird.

3. Verfahren nach einem der vorstehenden Ansprüche, **dadurch gekennzeichnet, dass** das Waschen des Roh-Dinitrotoluols in Schritt b) so erfolgt, dass zunächst neutrales, dann alkalisches und zuletzt neutrales Waschwasser verwendet wird.

4. Verfahren nach einem der vorstehenden Ansprüche, **dadurch gekennzeichnet, dass** die Abtrennung in Schritt c) in einem Lagertank mit Scheidevorrichtung vollzogen wird.

5. Verfahren nach einem der vorstehenden Ansprüche, **dadurch gekennzeichnet, dass** im Abgaskondensator in Schritt g) ein absoluter Druck im Bereich von 10 mbar bis 1200 mbar, bevorzugt von 100 mbar bis 1150 mbar eingestellt wird.

6. Verfahren nach einem der vorstehenden Ansprüche, **dadurch gekennzeichnet, dass** im Abgaskondensator in Schritt g) eine Temperatur von 20 °C bis 75 °C, bevorzugt von 25 °C bis 60 °C, und besonders bevorzugt von 30 °C bis 45 °C eingestellt wird.

7. Verfahren nach einem der vorstehenden Ansprüche, **dadurch gekennzeichnet, dass** das in Schritt g) auskondensierte Toluol der Nitrierung in Schritt a) zugeführt wird.

8. Verfahren nach einem der vorstehenden Ansprüche, **dadurch gekennzeichnet, dass** der dem Abgaskondensator zuzuführende oder der den Abgaskondensator verlassende Abgasstrom von Stickoxiden befreit wird.

9. Verfahren nach einem der vorstehenden Ansprüche, **dadurch gekennzeichnet, dass** die Schwefelsäure-haltige wässrige Phase nach der Abtrennung in Schritt a) gereinigt sowie optional aufkonzentriert und/ oder rezykliert wird.

## Claims

1. Process for producing dinitrotoluene, comprising the steps of:
a) nitration of toluene with a mixture of nitric acid and sulfuric acid with subsequent separation of a sulfuric-acid-containing aqueous phase formed during the nitration to obtain a crude dinitrotoluene,
b) washing the crude dinitrotoluene in a water wash with neutral and/or alkaline washing water, wherein after separation of the washing water employed in the last wash a prepurified dinitrotoluene is obtained which comprises besides dinitrotoluene also at least water and
c) separating the water from the prepurified dinitrotoluene,
d) collecting the wastewaters from steps a), b) and/or c),
e) optional extraction of the collected wastewaters from step d) with toluene and recycling of the thus obtained organic phase into step a),
f) freeing the collected wastewaters from step d) or, if optional step e) is performed, the extracted wastewater from step e) of toluene in a toluene stripper to obtain a toluene-containing offgas stream,
g) supplying the offgas stream from step f) into an offgas condenser and removing the toluene present in the offgas stream in this offgas condenser, wherein all offgas streams from steps a), b) and c) and also optionally d) and optionally e) are combined and fed either into the offgas stream that is to be supplied to the offgas condenser or into the offgas stream leaving the offgas condenser,
**characterized in that** the process comprises the further step of:
h) supplying the offgas stream generated in step g) after the condensing-out of the toluene to a thermal exhaust air purification, wherein nitrogen is added to the offgas stream to be supplied to the offgas condenser or to the offgas stream leaving the offgas condenser, wherein preferably a nitrogen concentration in the offgas stream of at least 0.1 vol% is established, particularly preferably of at least 0.5 vol%.

2. Process according to any of the preceding claims, **characterized in that** the nitration of toluene in step a) is performed in a continuous, two-stage process, wherein in the first stage a crude mononitrotoluene is obtained which in the second stage is converted into the crude dinitrotoluene.

3. Process according to any of the preceding claims, **characterized in that** the washing of the crude dinitrotoluene in step b) is effected such that initially neutral, then alkaline and finally neutral washing water is employed.

4. Process according to any of the preceding claims, **characterized in that** the separation in step c) is accomplished in a storage tank comprising a separator.

5. Process according to any of the preceding claims, **characterized in that** an absolute pressure in the range from 10 mbar to 1200 mbar, preferably from 100 mbar to 1150 mbar, is established in the offgas condenser in step g).

6. Process according to any of the preceding claims, **characterized in that** a temperature of 20°C to 75°C, preferably of 25°C to 60°C and particularly preferably of 30°C to 45°C is established in the offgas condenser in step g).

7. Process according to any of the preceding claims, **characterized in that** the toluene condensed out in step g) is supplied to the nitration in step a).

8. Process according to any of the preceding claims, **characterized in that** the offgas stream to be supplied to the offgas condenser or the offgas stream leaving the offgas condenser is freed of nitrogen oxides.

9. Process according to any of the preceding claims, **characterized in that** after the separation in step a) the sulfuric-acid-containing aqueous phase is purified and optionally concentrated and/or recycled.

## Revendications

1. Procédé de fabrication de dinitrotoluène, comprenant les étapes consistant à :
a) nitrer du toluène avec un mélange d'acide nitrique et d'acide sulfurique, puis séparer une phase aqueuse contenant de l'acide sulfurique formée lors de la nitration, un dinitrotoluène brut étant obtenu,
b) laver le dinitrotoluène brut dans un lavage à l'eau avec une eau de lavage neutre et/ou alcaline, un dinitrotoluène prépurifié, qui contient au moins de l'eau en plus du dinitrotoluène, étant obtenu après la séparation de l'eau de lavage utilisée dans le dernier lavage, et
c) séparer l'eau du dinitrotoluène prépurifié,
d) rassembler les eaux résiduaires des étapes a), b) et/ou c),
e) éventuellement, extraire les eaux résiduaires rassemblées de l'étape d) avec du toluène et recycler la phase organique ainsi obtenue dans l'étape a),
f) débarrasser les eaux résiduaires rassemblées de l'étape d) ou, si l'étape e) optionnelle est réalisée, l'eau résiduaire extraite de l'étape e), du toluène dans un épuiseur de toluène, un courant de gaz d'échappement contenant du toluène étant obtenu,
g) introduire le courant de gaz d'échappement de l'étape f) dans un condensateur de gaz d'échappement et éliminer le toluène contenu dans le courant de gaz d'échappement dans ce condensateur de gaz d'échappement, tous les courants de gaz d'échappement des étapes a), b) et c), ainsi qu'éventuellement d) et éventuellement e), étant réunis et introduits soit dans le courant de gaz d'échappement à introduire dans le condensateur de gaz d'échappement, soit dans le courant de gaz d'échappement quittant le condensateur de gaz d'échappement,
**caractérisé en ce que** le procédé comprend l'étape supplémentaire consistant à :
h) introduire le courant de gaz d'échappement formé à l'étape g) après la condensation du toluène dans une purification de l'air d'échappement thermique, de l'azote étant ajouté dans le courant de gaz d'échappement à introduire dans le condensateur de gaz d'échappement ou dans le courant de gaz d'échappement quittant le condensateur de gaz d'échappement, une concentration d'azote dans le courant de gaz d'échappement d'au moins 0,1 % en volume étant de préférence ajustée, de manière particulièrement préférée d'au moins 0,5 % en volume.

2. Procédé selon l'une quelconque des revendications précédentes, **caractérisé en ce que** la nitration de toluène à l'étape a) est réalisée dans un procédé continu à deux étapes, un mononitrotoluène brut étant obtenu dans la première étape, qui est transformé en le dinitrotoluène brut dans la deuxième étape.

3. Procédé selon l'une quelconque des revendications précédentes, **caractérisé en ce que** le lavage du dinitrotoluène brut à l'étape b) a lieu en utilisant tout d'abord une eau de lavage neutre, puis alcaline et en dernier neutre.

4. Procédé selon l'une quelconque des revendications précédentes, **caractérisé en ce que** la séparation à l'étape c) est exécutée dans une cuve de stockage munie d'un dispositif de coupe.

5. Procédé selon l'une quelconque des revendications précédentes, **caractérisé en ce qu'**une pression absolue dans la plage allant de 10 mbar à 1 200 mbar, de préférence de 100 mbar à 1 150 mbar, est ajustée dans le condensateur de gaz d'échappement à l'étape g).

6. Procédé selon l'une quelconque des revendications précédentes, **caractérisé en ce qu'**une température de 20 °C à 75 °C, de préférence de 25 °C à 60 °C, et de manière particulièrement préférée de 30 °C à 45 °C, est ajustée dans le condensateur de gaz d'échappement à l'étape g).

7. Procédé selon l'une quelconque des revendications précédentes, **caractérisé en ce que** le toluène condensé à l'étape g) est introduit dans la nitration à l'étape a).

8. Procédé selon l'une quelconque des revendications précédentes, **caractérisé en ce que** le courant de gaz d'échappement à introduire dans le condensateur de gaz d'échappement ou quittant le condensateur de gaz d'échappement est débarrassé des oxydes d'azote.

9. Procédé selon l'une quelconque des revendications précédentes, **caractérisé en ce que** la phase aqueuse contenant de l'acide sulfurique est purifiée et éventuellement concentrée et/ou recyclée après la séparation à l'étape a).
